Europäisches Patentamt

European Patent Office

Office européen des brevets

(19)

(11) Publication number: **0 386 692**

**A2**

(12) # EUROPEAN PATENT APPLICATION

(21) Application number: **90104253.1**

(22) Date of filing: **06.03.90**

(51) Int. Cl.5: **B01J 8/04, C01C 1/04, C07C 29/15**

(30) Priority: **09.03.89 CH 873/89**

(43) Date of publication of application:
**12.09.90 Bulletin 90/37**

(84) Designated Contracting States:
**AT DE ES FR GB GR IT NL**

(71) Applicant: **AMMONIA CASALE S.A.**
**Via della Posta 4**
**CH-6900 Lugano(CH)**

Applicant: **Zardi, Umberto**
**Via Lucino 57**
**CH-6932 Breganzona (Ti)(CH)**

(72) Inventor: **Zardi, Umberto**
**Via Lucino 57**
**CH-6932 Breganzona(CH)**
Inventor: **Pagani, Giorgio**
**Salita dei Frati 13**
**CH-6900 Lugano(CH)**

(74) Representative: **Incollingo, Italo**
**AMMONIA CASALE S.A. Via della Posta 4**
**CH-6900 Lugano(CH)**

(54) **System for modifying in situ reactors for the synthesis of ammonia.**

(57) The system for modifying in situ an existing reactor with axial flow into a reactor with substantially radial flow with three catalytic beds, at least one heat exchanger in one of the two lower beds, and at least a quenching device in the upper bed, consists in: installing at the bottom of the pre-emptied shell the catalytic basket free of any catalyst but with a central exchanger already built in; filling of the catalyst forming the lower bed; installing the intermediate basket free of any catalyst; inserting in it the heat exchanger; loading the catalyst forming the second bed; installing the third upper basket with its ring for the distribution of quench gas; and filling this basket with the catalyst forming the third upper bed.

At the disassembly stage, stripping operations are substantially in the reverse order and are preceded by nitrogen flooding in the various catalytic beds.

## System for modifying in situ reactors for the synthesis of ammonia

This invention concerns a system for modernizing in situ reactors for the synthesis of ammonia, methanol and similar materials, having an outer shell, an inner cartridge, several catalytic beds, heat exchangers, as well as a quenching system.

In a series of previous patents by the Applicants (for example US Patents No. 4,372,920 and 4,405,562) new low energy consumption reactors were described, with a substantially radial flow, and more particularly with an axial-radial flow. In US Patent No. 4,755,362 a system was described to transform in situ old high energy consumption reactors with an axial flow into essentially radial reactors.

In recent Swiss patent applications optimized reactors were described, achieved through in situ conversion and characterized by the fact that:
- the cartridge-forming shell is substantially independent of the catalytic baskets;
- at least a first end basket rests on an intermediate basket which in turn rests on a packing ring integral with said cartridge shell, the bottoms of said two coupled baskets being inversely curved with respect to the curve at the bottom of the reactor;
- in said first end bed a collector is placed between the basket's outer wall and the internal face of the cartridge shell;
- in said intermediate bed a heat exchanger or a quench collector is placed inside the internal cylindrical wall forming the basket; and
- a gas/gas heat exchanger is placed in a central position inside the cylindrical wall of at least the catalytic basket closest to the reactor outlet for hot reacted gas.

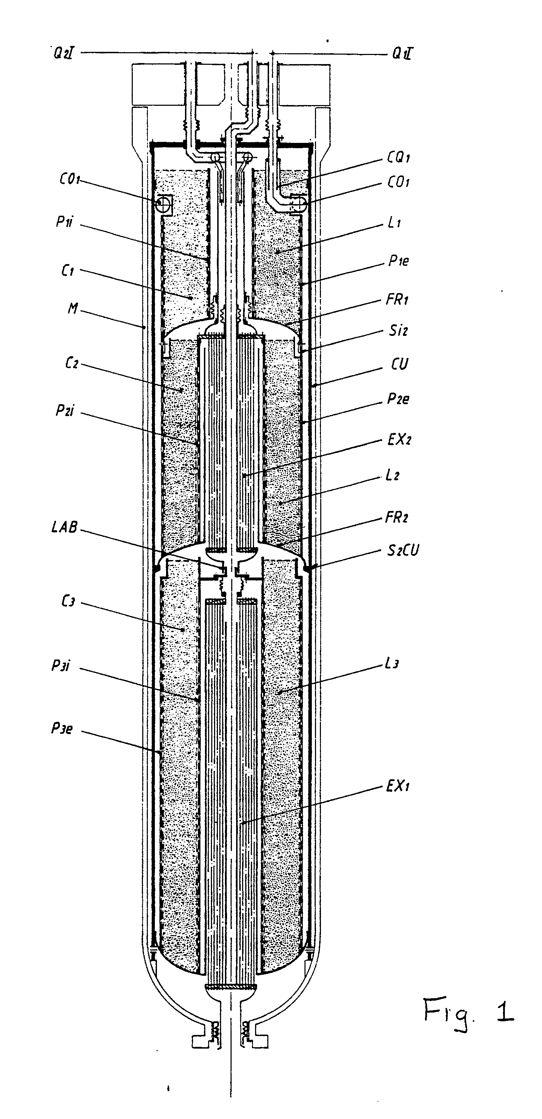

In order to fix ideas clearly and straight away, in figure 1 a preferred embodiment is shown of said reactors; M indicates the outer shell; CU the cartridge; L1, L2, L3 the three catalytic beds, each delimited by substantially perforated outer walls P1e, P2e, P3e and internal walls P1i, P2i, P3i; CQ1 (and possibly CQ2) is the duct feeding quench gas Q1 (Q2) to collectors CO1 (CO2); EX1 and EX2 the gas/gas heat exchangers in lower bed L3, respectively in intermediate bed L2; Q1I (and possibly Q2I) the inlets for quench gas, respectively synthesis gas; CO1 (and possibly CO2) the collectors-distributors of quench gas; S12 the support for bed L1 at the top of bed L2 and S2 CU the support for L2 on a ring of cartridge CU.

Continuing their research and experiments (above all in situ), the Applicants have now perfected a system for optimizing the conversion on the spot of existing reactors into highly efficient reactors with low energy consumption, for example of the type shown (not by way of limitation) in figure 1.

The invention concerns in its general form a system for assembling and disassembling cartridges and catalytic beds; it concerns more particularly also a system for disassembling the cartridge in the event of breakdown and/or maintenance very efficient for keeping the catalyst in the catalytic beds in situ, without its being damaged thus reducing its activity.

The importance of being able to install in the shortest possible time the main equipment of chemical plants such as for example synthesis reactors is well known, and this in order to achieve the efficient operation of the equipment in the shortest possible time thus reducing standstill time.

In the particular case of the synthesis reactor cartridge shown in figure 1, cartridge CU installed inside the shell consists of the following elements:
1) 3 catalytic beds (L1, L2, L3);
2) 2 exchangers EX1 and EX2 coupled together for example by means of a labyrinth seal (LAB);
3) 1 quench ring (CO1);
4) catalyst (C1, C2, C3) installed inside catalytic beds (L1, L2, L3).

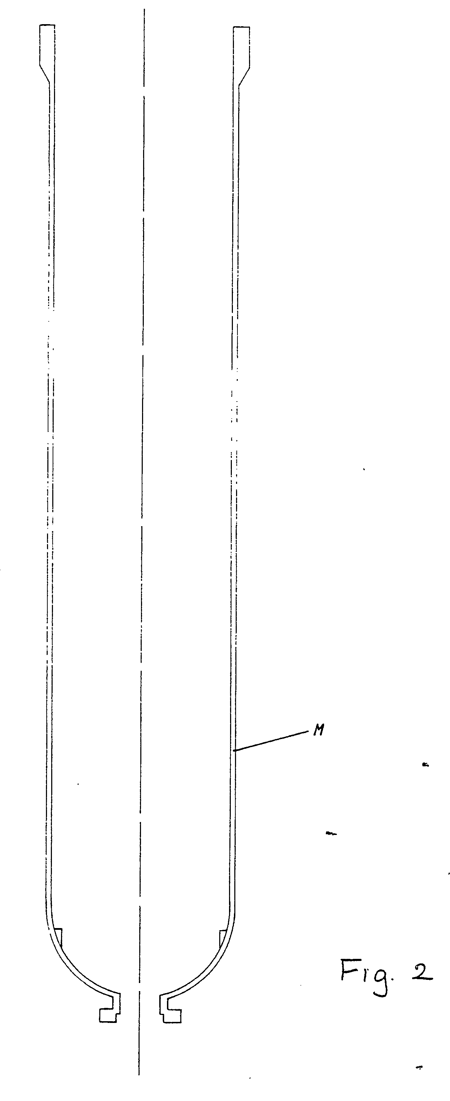

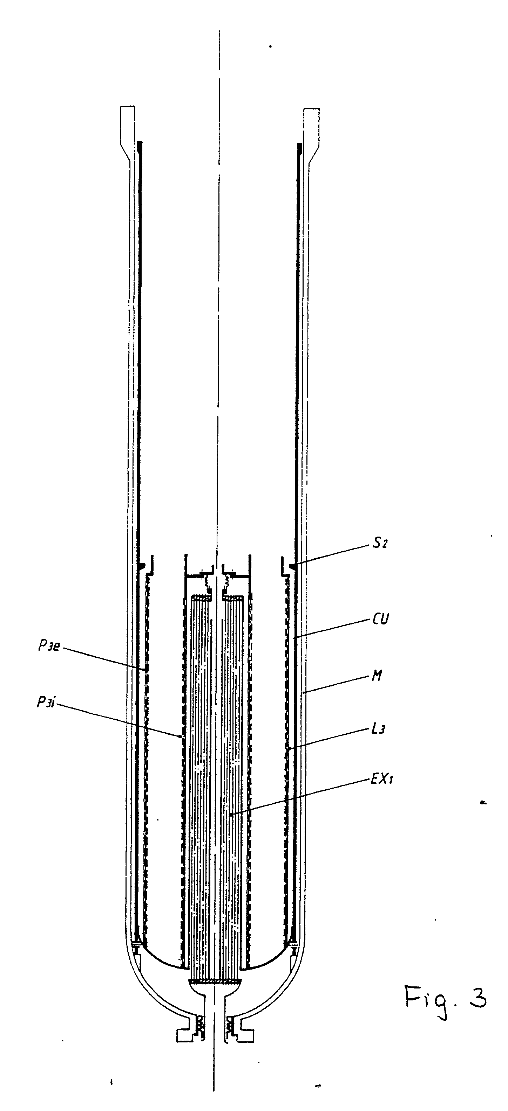

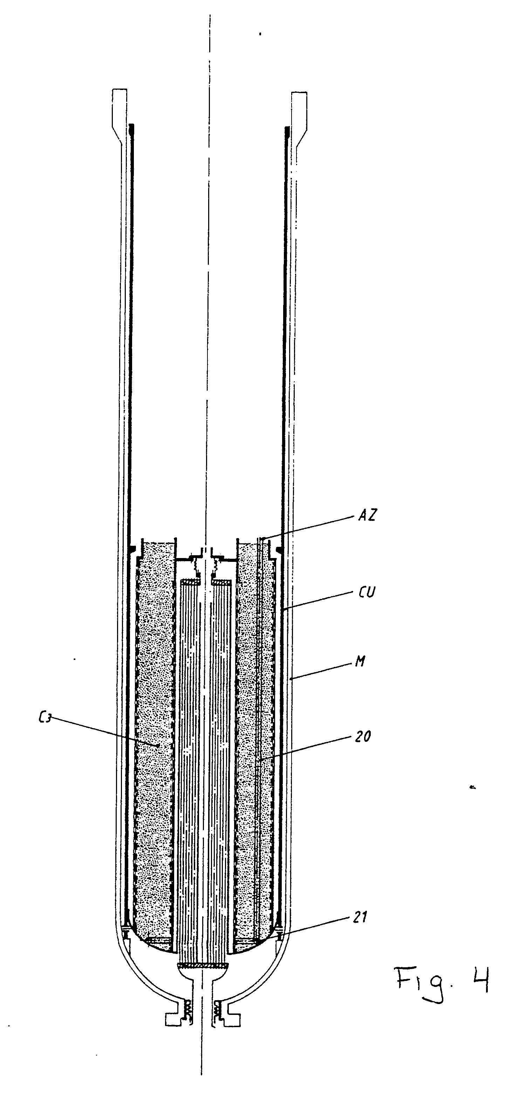

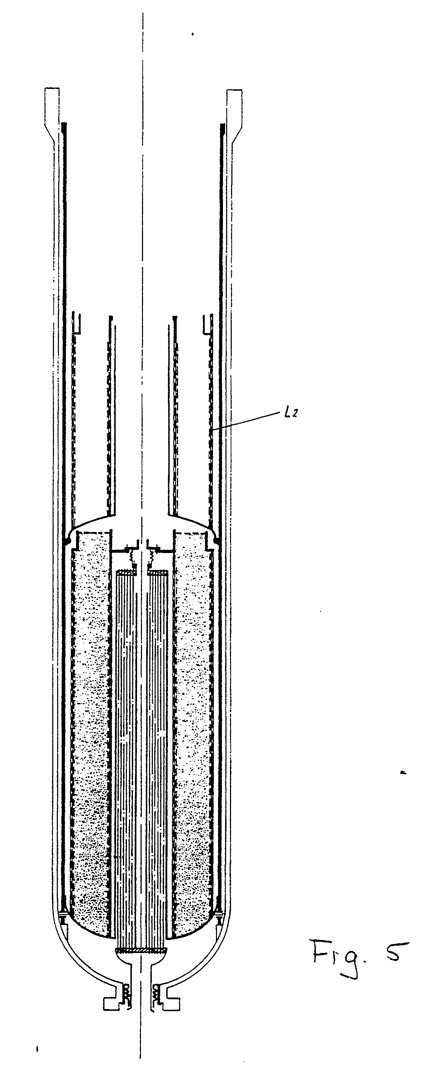

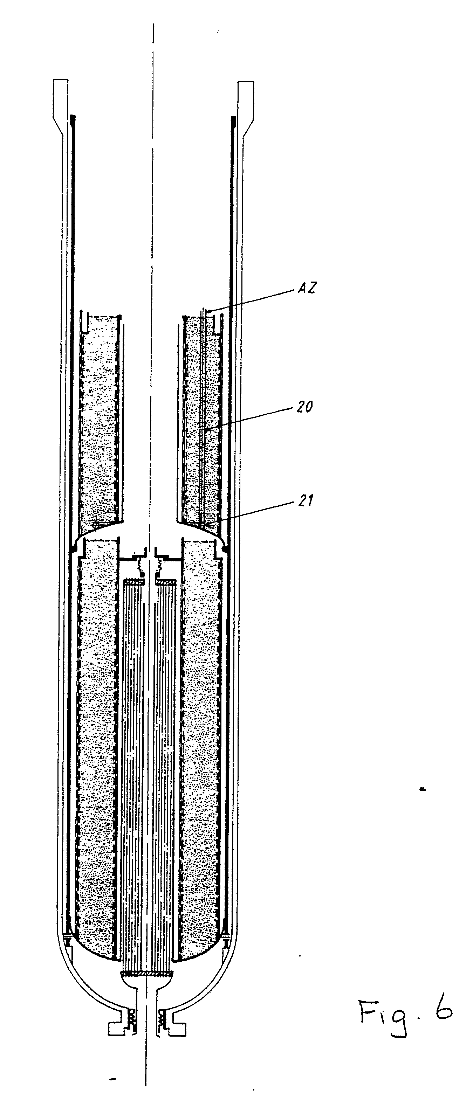

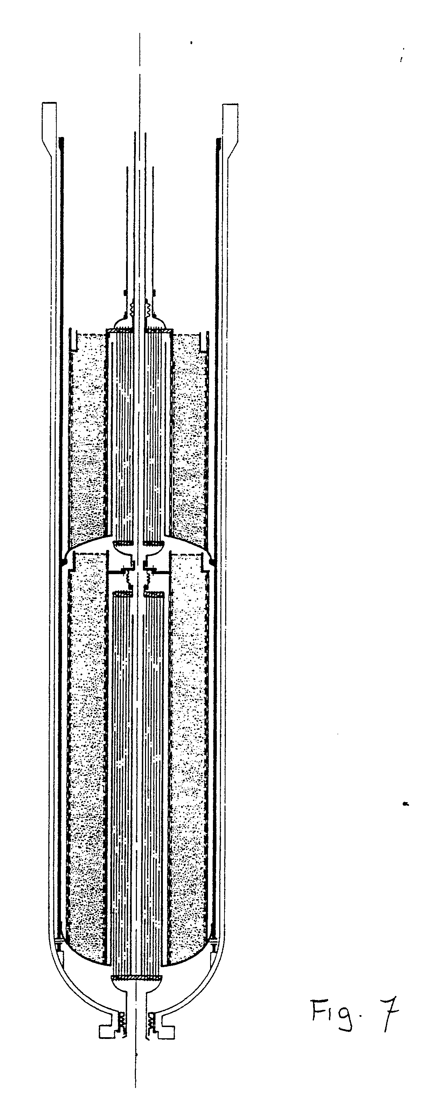

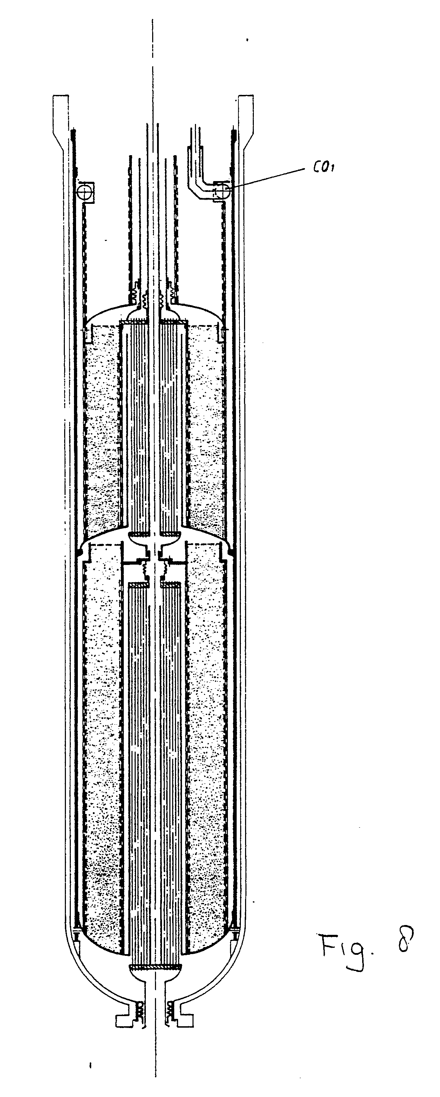

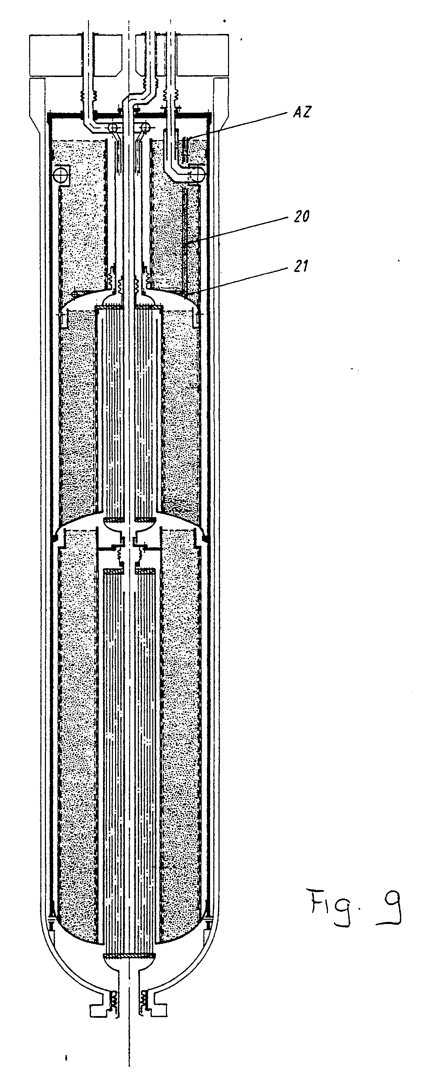

According to the system which is the subject of this invention the assembly procedure consists of the following steps:
1. Installation, inside the emptied shell M (figure 2), of the empty lower bed L3 and its exchanger EX1 inserted in bed L3 (figure 3).
2. Insertion of catalyst C3 inside lower bed L3 (figure 4). Possible creation of a protective gaseous atmosphere by feeding nitrogen AZ from a container in duct 20 which has at its lower end a drilled torispherical pipe 21 for gas distribution.
3. Installation of empty intermediate bed L2 (figure 5), by resting its feed on support S2 projecting from the internal face of cartridge CU.
4. Installation in intermediate bed L2 of catalyst C2 (figure 6). Possible protection with nitrogen L3.
5. Installation of second exchanger EX2 (figure 7).
6. Installation of upper bed L1 with its quench ring CO1 (figure 8).
7. Installation of catalyst L1 inside the upper bed (figure 9).

As an advantageous alternative, in order to further reduce installation time, catalyst C2 in intermediate bed L2 and catalyst C1 in upper bed L1 can be installed at the same time and outside shell M (installation of catalytic beds, already loaded with catalyst, inside the shell).

Preferably the optimal procedure for disassembling capable of reducing standstill time and if necessary maintain the catalyst undamaged (no loss of activity) is as follows:

1. After opening the basket by removing the upper lid, nitrogen AZ is flooded inside the catalyst in the first catalytic bed L1. A suitable nitrogen AZ flooding system if provided for inside bed L1 consisting for example of a duct 20" and of a drilled torispherical pipe 21" for gas distribution (figure 9).

2. Catalytic bed L1 containing catalyst C1 is removed and nitrogen flooding is carried on, while outside the bed suitable housing is temporarily installed so as to avoid nitrogen dispersion maintaining the bed in a pure nitrogen atmosphere.

3. A similar procedure is adopted for intermediate catalytic bed L2, preferably in a nitrogen atmosphere (figure 6).

4. Bottom bed L3 is kept inside the shell, flooding nitrogen inside the catalytic bed as in the preceding beds (figure 9).

## Claims

1. System for assembling and disassembling catalytic cartridges in ammonia or methanol reactors, and more particularly during the conversion "in situ" of existing reactors with axial flow and high energy consumption into low energy consumption reactors with a substantially radial flow, each reactor consisting specifically of a shell and a catalyst-holding cartridge, three superimposed catalytic baskets, at least a heat exchanger and at least a quenching system, characterized by the fact that at the assembly stage at the bottom of the empried existing shell (M) is installed the empty lower catalytic basket (L3) with its heat exchanger (EX1) inserted centrally; the catalyst (C3) is loaded inside the perforated walls of the basket (L3); the intermediate empty catalytic basket (L2) is installed; the heat exchanger (EX2) is installed centrally to (L2); the catalyst (C2) is loaded between the internal and external walls of said basket (L2); the third empty basket (L1) with its quench gas distribution ring (CO1) is then installed; and catalyst (C1) is loaded filling the annular zone between the external and internal walls of said basket (L1).

2. System according to claim 1 in which loading of the catalyst (C2, respectively C1) in the intermediate (L2) and/or upper (L1) baskets takes place outside the shell.

3. System according to claim 1, characterized by the fact that at the disassembly stage nitrogen is flooded into the catalyst (C1) of the upper bed (L1); this basket (L1) is removed together with its catalyst; nitrogen is flooded into the catalyst of the second intermediate bed and the basket is then removed with its catalyst; the third basket is kept inside the shell after having flooded with nitrogen the catalyst it contains.

Fig. 1

Fig. 2

Fig. 3

Fig. 4

L2

Fig. 5

AZ

20

21

Fig. 6

Fig. 7

$CO_1$

Fig. 8

Fig. 9